Europäisches Patentamt

European Patent Office

Office européen des brevets

(11) Publication number: **0 296 413**
**A2**

## EUROPEAN PATENT APPLICATION

(21) Application number: **88109186.2**

(22) Date of filing: **09.06.88**

(51) Int. Cl.⁴: **C12N 15/00 , C12N 9/64**

The applicant has filed a statement in accordance with Rule 28 (4) EPC (issue of a sample only to an expert). Accession number(s) of the deposit(s): FERM BP-1858, FERM BP-1473, FERM BP-1472.

Claims for the following Contracting States: ES + GR.

(30) Priority: **12.06.87 JP 145293/87**

(43) Date of publication of application:
**28.12.88 Bulletin 88/52**

(84) Designated Contracting States:
**AT BE CH DE ES FR GB GR IT LI LU NL SE**

(71) Applicant: **HOECHST JAPAN LIMITED**
**10-16, 8-chome, Akasaka, Minato-ku**
**Tokyo(JP)**

(72) Inventor: **Iwasaki, Wakako**
**Dohkan-Haitsu 405 3-9-1 Kamiigusa**
**Suginami-Ku Tokyo(JP)**
Inventor: **Takahashi, Mikiko**
**1-11, Ayase**
**Hasuda-shi Saitama-ken(JP)**
Inventor: **Hashimoto, Tamotsu**
**1-5-10-309, Sakae-Cho**
**Asaki-Shi Saitama-Ken(JP)**

(74) Representative: **Klein, Otto, Dr. et al**
**Hoechst AG Zentrale Patentabteilung**
**Postfach 80 03 20**
**D-6230 Frankfurt am Main 80(DE)**

(54) **Hybrid protein C and method for its preparation.**

(57) A hybrid protein of human protein C whose Gla domain is replaced by an amino acid sequence containing Gla domain of human vitamin K dependent proteins which participate in blood coagulation or by its equivalent is disclosed. Examples of vitamin K dependent proteins are human prothrombin and Factor VII, Factor IX, and Factor X.

The hybrid protein is produced by culturing host cells transfected with a plasmid vector containing DNA coding it. DNA corresponding to Gla domain to replace can be easily synthesized and ligated with DNA coding protein C downstream of the Gla domain.

EP 0 296 413 A2

## Hybrid Protein C and Method for its Preparation

Detailed Explanation of the Invention

(1) Applied fields in industry:

This invention relates to novel hybrid protein C having anti-blood coagulation activity.

(2) Technological background:

Human protein C is a precursor of a serine protease detected in human plasma.

It is well known that human protein C is activated by a thrombin-thrombomodulin complex. Activated protein C inhibits blood coagulation through inactivation of Factor VIIIa and Factor Va in the presence of calcium ions.

The gene corresponding to expression of human protein C was cloned and sequenced by Foster et al., Proc. Natl. Acad. Sci. USA, 82, 4673-4677, (1985). The amino acid sequence of human protein C was also determined from the nucleotide sequence coding for human protein C by Foster et al., ibid.

Human protein C has 9 characteristic glutamic acid residues at the 6th, 7th, 14th, 16th, 19th, 20th, 25th, 26th, and 29th positions in the amino terminal region. These glutamic acid residues are carboxylated at their $\gamma$-carbon through vitamin K dependent post-translational modification. The domain including these $\gamma$-carboxyl glutamic acid residues (hereinafter referred to as "Gla domain") is required for complex formation in the presence of calcium with negatively charged phospholipids on the cell membrane. The function of Gla domain was reviewed in Taisha (Metabolism), 19, No. 9 (1982) (in Japanese).

Besides protein C, several other vitamin K dependent human blood proteins having a similar Gla domain structure but with a reversed pro-coagulation effect, i.e., prothrombin, Factor VII, Factor IX, and Factor X, have been well investigated so far.

Recently interesting information was reported by Nawroth et al., Thrombosis Research, 44, 625-637 (1986), that the peptide covering the 1st to 43rd amino acid residues from amino terminal of Factor X inhibits competitively Factor X activity and, in turn, suppresses blood coagulation.

(3) Purpose of the invention:

Purpose of this invention is to supply novel hybrid proteins having more potent anti-coagulation activity constructed by replacing a segment of human protein C with a corresponding segment from the other vitamin K dependent coagulation proteins.

(4) Construction of the invention:

The present invention is directed to hybrid proteins constructed by replacing the Gla domain of human protein C with the Gla domain of the other vitamin K dependent proteins responsible for human blood coagulation or with its equivalent. Prothrombin, Factor VII, Factor IX, and Factor X have been known as vitamin K dependent proteins concerning human blood coagulation system.

The amino acid sequences near the amino terminal of each vitamin K dependent protein are listed in Table 1, aligned to maximize the homology among the corresponding amino acid residues by considering the physicochemical homology based on the definition of Dayhoff (1978).

The amino acid residues in Table 1 are abbreviated to the following:
A, Ala; N, Asn; S, Ser; F, Phe; L, Leu; E, Glu; R, Arg; H, His; C, Cys; I, Ile; D, Asp; K, Lys; Q, Gln; V, Val; P, Pro; T, Thr; W, Trp; M, Met; G, Gly; Y, Tyr
* designates a nonsense codon.
The glutamic acid residues, designated as E in this table, are $\gamma$-carboxylated.

In this invention, the amino acid sequence from the 1st to 43rd residues of human protein C is substituted with an amino acid sequence from the lst to 43rd residues of human prothrombin. Factor VII. Factor IX, or Factor X.

It is possible to replace one or more amino acid residues with other one or more amino acid residues as long as the activity of constructed hybrid protein is not deteriorated.

The hybrid proteins in this invention can be produced by genetic engineering techniques. Therefore, this invention is also concerned with the DNA coding for these hybrid proteins and the method of producing these hybrid proteins through genetic engineering techniques.

The gene coding for hybrid proteins in this invention can be constructed by replacing the nucleotide sequence coding the amino acid sequence including Gla domain and the leader sequence of the gene for human protein C with the nucleotide sequence coding the amino acid sequence including Gla domain and the leader sequence of vitamin K dependent blood coagulation proteins. In this case it is also possible to ligate the nucleotide sequence coding Gla domain of Factor IX with the leader sequence of Factor X.

The nucleotide sequence coding protein C was published in the above mentioned Foster's reference and in the gazette, Japanese Laid-Open Unexamined Patent Publication 205487/86.

The nucleotide sequences of genes coding for prothrombin, Factor VII, Factor IX, and Factor X had been published in Biochemistry, 22, 2078 (1983), Proc. Natl. Acad. Sci. USA, 83, 2412 (1986), Proc. Natl. Acad. Sci. USA, 79, 6461 (1982) and Biochemistry, 25, 5098 (1986), respectively.

In the present invention, it is preferable to synthesize the nucleotide sequences coding the amino acid sequences including Gla domain and leader sequences according to these nucleotide sequences published in the above listed references.

The genes constructed by the above methods can be expressed in the proper host cells transformed by the proper expression vectors constructed by ligating the protein coding sequence with a promoter, a terminator, etc. by the ordinary methods in this field. Preferable hosts are eukaryotic cells, which can modify the recombinant proteins in post-translational manners, e.g., glycosylation, $\gamma$-carboxylation or $\beta$-hydroxylation. Preferable promoters for eukaryotic cells are SV40 early promoter or mouse mammary tumor virus, etc.

Examples for host cells favorable for production are animal cells such as CHO, 8HK, HEK or HepG2 cells. The construction of recombinant genes, the additional ligation of functional regions necessary for efficient expression, transfection to host cells, expression of recombinant products in host cells, and isolation of recombinant products, etc. are possible to do by any methods generally available in this field.

Hybrid proteins to be produced in this invention are expected to have competitive inhibitory activity against vitamin K dependent blood coagulation proteins in addition to the neutralization activity against plasminogen activator inhibitor and inactivation of Factor Va and Factor VIIIa, which are original functions of protein C. Therefore, the hybrid proteins disclosed in this invention have better activity against blood clot formation or fibrinolysis accelerating effect.

As examples, a gene coding human protein C newly prepared and a new expression method were employed. A brief explanation of them is given in the following.

The inventors constructed a human protein C-coding gene consisting of 1389 bp of nucleotides and additionally having EcoRI site at one end, and SmaI and HindIII sites at the other end. The gene was prepared on the basis of the nucleotide sequence coding human protein C published by Foster et al., ibid., with some changes of bases without changing amino acid residues.

Plasmid pPCI was formed by integrating the gene between EcoRI and HindIII of the plasmid vector which was derived from pUC9 (commercially available from Pharmacia) and had the same polylinker site as in pUC9 and an ampicillin-resistant gene with PvuI lost. The plasmid was transformed into E. coli K-12 Om225 which was deposited with the Fermentation Research Institute, Agency of Industrial Science and Technology as FERM BP-1858.

Furthermore, an expression vector pCsI was constructed from the gene. Namely, the plasmid pSVAstopI, in which a fragment containing SV40 early promoter and a fragment containing a polylinker for insertion of the gene and SV40 early mRNA polyadenylation site were connected to 2.3 Kbp PvuII-EcoRI fragment of the plasmid pBR322 (which contains an ampicillin-resistant gene and a duplication initiation site), was cleaved at XbaI site presesnt in the polylinker and formed to a flush end with T₄ DNA polymerase. Then, the pPCI was digested with EcoRI and SmaI, and 1.4 Kbp DNA fragment containing a protein C gene was isolated and purified by polyacrylamide gel electrophoresis and converted to a flush end with T₄ DNA polymerase, which was then ligated with T₄ DNA ligase to the flush end of the plasmid pSVAstopI.

The recombinant DNA plasmids were examined to select a clone in which a protein C gene was integrated in the orientation expressible under the SV40 early promoter. The selected clone was named pCsI. The plasmid was transformed into E. coli K-12 HB101 which was deposited with the Fermentation

3

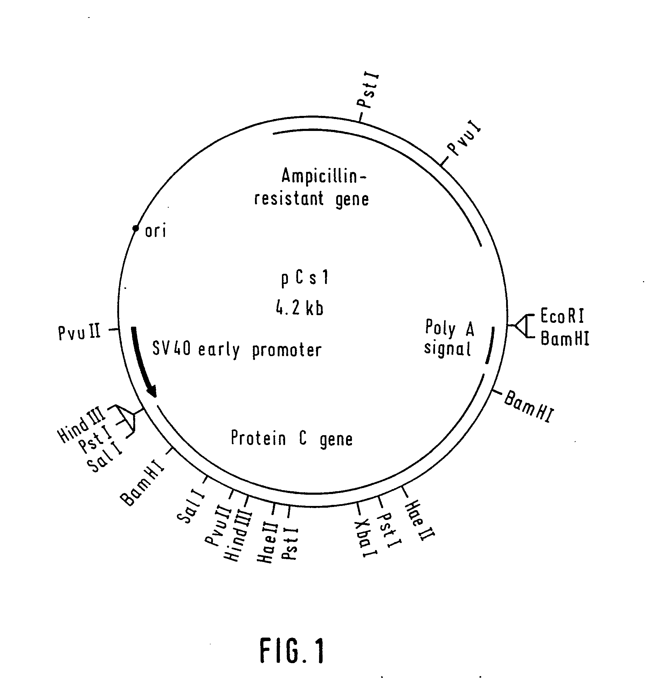

Research Institute as FERM BP-1473. The restriction map is shown in Fig. 1.

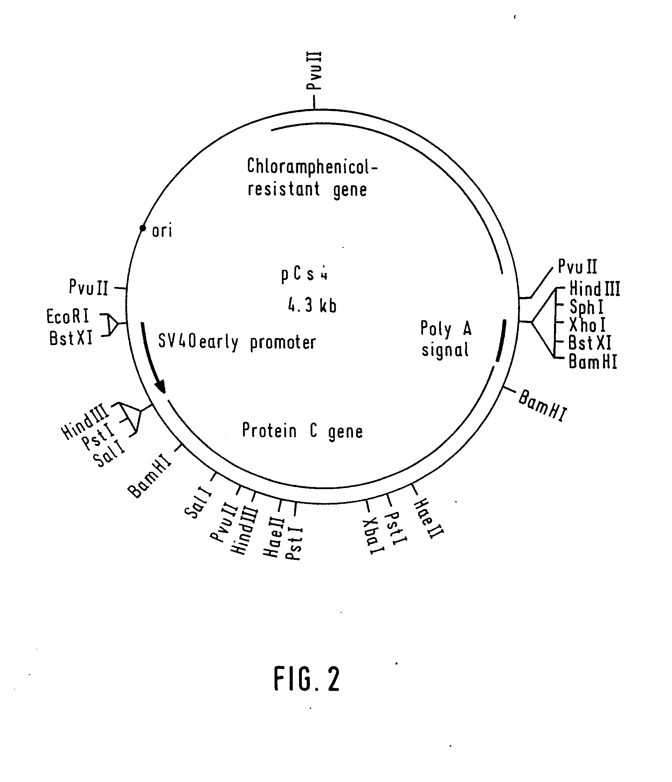

pCs4 was constructed from pCs1. pCs4 has two BstXI sites upstream from SV40 early promoter and downstream from poly A signal as illustrated in Fig. 2. The BstXI site was devised to produce the following asymmetric cohesive ends by digestion with BstXI.

$$5' \cdots\cdots CTGG \boxed{Gene} CCACGGGG \cdot 3'$$
$$3' \cdot CCCCGACC \phantom{\boxed{Gene}} GGTG \cdots\cdots 5'$$

The fragments link with each other necessarily in tandem. This makes it possible to prepare DNA comprising many tandem repeats of expression unit of protein C (a promoter plus a protein C gene plus a poly A signal). Protein C is produced by cultivating host animal cells transfected with the DNA comprising many tandem repeats. Production process of pCs4 is as follows: the fragment obtained by digesting pCs1 with EcoRI was ligated with the double-stranded oligonucleotide synthesized chemically as mentioned below.

$$5' \text{ pAATTG } \boxed{CCA} \text{ CGGGGC } \boxed{TGG} \text{ C}\cdots\cdots 3'$$
$$3' \cdots\cdots\text{C } \boxed{GGT} \text{ GCCCCG } \boxed{ACC} \text{ GAGCTp } 5'$$

wherein p represents the phosphate group coupled at the 5′ ends for the ligation, ▭ indicates the BstXI recognition site, and indicates the cleavage site by the said enzyme.

The left side of the oligonucleotide is arranged so as to a 5′ protruding sequence which can be ligated to the cohesive end of EcoRI-cleaved pCs1, which does not make the EcoRI site regenerate. This arrangement is made so that the EcoRI site produced in the subsequent step will a unique site. The right side is the portion that is linked with the XhoI cohesive end.

The ligation product was digested with XhoI followed again by the ligation. Both ends of the pCs1 cleaved with EcoRI were linked respectively with one molecule of the synthetic oligonucleotide. Both ends of the product were subjected to the ligation (wherein XhoI site is formed) to form a circular plasmid.

Then, the resulting plasmid was partially digested with PvuII. As there were two PvuII sites in pCs1, the cleavage took place at both, either one or none of the sites to give a mixture of them. Accordingly, the mixture was subjected to size fractionation by means of the electrophoresis using agarose gel to isolate the product in which the PvuII located upstream of SV40 early promoter only had been cleaved. The isolated DNA chain was ligated with a chemically synthesized double-stranded oligonucleotide with the chemical structure shown below.

$$5' \text{ pTT } \boxed{CCA} \text{ GCCCCG } \boxed{TGG} \cdots\cdots 3'$$
$$3' \cdot\text{AA } \boxed{GGT} \text{ CGGGGC } \boxed{ACC} \text{ TTAAp } 5'$$

wherein symbols are as defined above, and the right side is the portion that is linked with the section cleaved by EcoRI.

The left side of the oligonucleotide was linked with the PvuII site of the cleaved plasmid DNA, which did not make the PvuII site regenerate. The ligation product was digested with EcoRI followed again by the ligation. Both ends of the PvuII-cleaved plasmid DNA were linked respectively with one molecule of the synthetic oligonucleotide. Both ends of the product were subjected to the ligation (wherein EcoRI site is located) to form a circular plasmid. The resulting plasmid was cleaved with XhoI and EcoRI, and the fragment was cloned into pHSG396 having a chloramphenicol-resistant marker (available from Takara Shuzo Co., Ltd.) cleaved with XhoI and EcoRI. The chloramphenicol-resistant protein C expression vector plasmid

4

thus obtained was named pCs4. Its restriction map is shown in Fig. 2.

pHSG293 was also constructed which had a neo gene as a selection marker and BstXI site providing the same asymmetric cohesive ends as pCs4 by digestion. The fragment obtained by digesting pHSG293 with BstXI was ligated with the fragment obtained by digesting pCs4 so that the selection of transfectants was possible. A production process is as follows: when pHSG274 deposited as ATCC 37301, a cosmid vector with the neo gene which confers kanamycin resistance to E. coli host and G418 (geneticin) resistance in eucaryotic cells was digested with BstXI, and cleavage took place as shown in the following flow sheet. Then a synthetic nucleotide shown in the flow sheet was ligated.

pHSG274
↓ BstXI

CCA TCATG         A TGG  ---- HindIII
GGT A        GTACT ACC

cos

Synthetic
Oligonucleotide GTAC CCA GCCCCG TGG —XbaI—lacOP— A
                 GGT CGGGGC ACC          TTCGA
( BstXI⁻ )       BstXI        (HindIII)

↓ Ligation

BstXI⁻ — CCA GCCCCG TGG —XbaI—lacOP— A
           GGT CGGGGC ACC         TTCGA
          BstXI          (HindIII)

cos

        A TGG ----- HindIII
    GTACT ACC

↓ HindIII

BstXI⁻ — CCA GCCCCG TGG —XbaI—lacOP— A
           GGT CGGGGC ACC         TTCGA
          BstXI          (HindIII)

cos

(HindIII)

↓ Ligation

pHSG 293

↓ BstXI

3.6kb fragment

The left side of the synthetic oligonucleotide was linked with the cosmid vector DNA cleaved by BstXI and the BstXI site was destroyed. Subsequent digestion of the cleavage product with HindIII results in removal of the HindIII-BstXI portion contained in the cosmid vector and the excessive synthetic fragments repeatedly ligated. The resulting product was subjected to ligation to afford a circular plasmid in which the right side of the synthetic oligonucleotide was linked with the HindIII site of the cosmid vector.

The XbaI site in the synthetic oligonucleotide sequence was introduced to distinguish pHSG274 and pHSG293 from each other. The lac operator in the synthetic oligonucleotide was introduced to select transformants by kanamycin resistance after cosmid-packaging and to distinguish transformants that form blue colonies in the presence of X-gal.

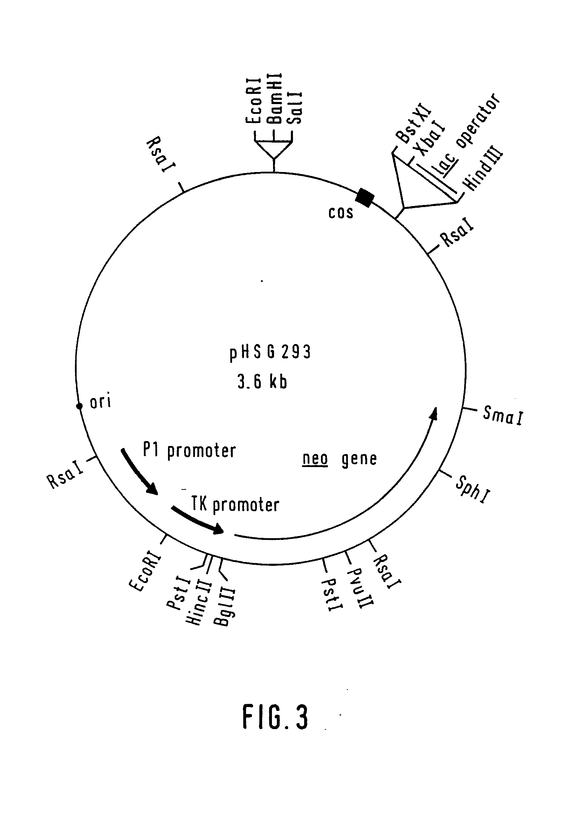

The plasmid was named pHSG293. Its restriction map is shown in Fig. 3.

In the following example, oligonucleotides covering leader sequences and Gla domain of vitamin K dependent blood coagulation proteins were synthesized. The nucleotide sequence coding the leader sequence and Gla domain of protein C in pCs4 was substituted by the synthetic oligonucleotides. The constructed gene units making a hybrid protein expressible and the neo gene removed from pHSG293 were multiply-linked to the same orientation by using uni-directed cohesive ends of BstXI.

Then, these multiple linked genes were in vitro packaged in phage particles and transfected into E. coli cells. The transfectants were screened to obtain clones harbouring desired recombinant cosmid DNAs through kanamycin resistance derived from the neo gene. Then, the recombinant cosmid DNAs obtained by the above method were introduced into CHO cells by an ordinary method available in this field. Again, G418-resistant CHO cells derived from the neo gene were screened, which efficiently produced desired hybrid proteins.

The hybrid proteins disclosed in this invention can be produced by any genetic engineering techniques. Therefore, the examples mentioned below are only one type of examples to produce hybrid proteins, which do not limit our invention to these examples.

## Example 1

### Synthesis of a DNA fragment coding an amino acid sequence including Gla domain of Factor X

A DNA fragment, the sequence of which is shown in Table 2, was synthesized so as to be flanked by SalI site and EcoRI site for convenience of ligation and to cover (a) the 40 amino acid residues of leader sequence of Factor X, (b) the 1st to 43rd amino acid residues from the N-terminus of mature Factor X following the leader sequence, and (c) the nucleotide sequence coding the 44th to 46th amino acid residues of human protein C. The adenine just before the start ATG was selected to expect a preferable effect on expression of hybrid proteins. And the nucleotide sequence coding the 44th to 46th amino acid residues of protein C was introduced to create a new SalI site to ligate the DNA fragment with the human protein C gene.

Eight oligonucleotides to yield four DNA fragments separated with HindIII, XbaI, and PstI, shown in Table 2, were synthesized by a DNA synthesizer, model 380A (Applied Biosystem Co., U.S.A.). After anealing, four double-strand fragments were ligated by T₄ DNA ligase at the HindIII, XbaI and PstI sites, respectively. The ligated DNA fragment flanked by SalI sites was inserted into pUC18, commercially available from Takara Shuzo Co., Ltd., and amplified in E. coli K-12/HB101. The amplified vector was digested by SalI, and the synthetic DNA fragment was obtained in medium amount by ordinary purification methods.

Essentially by the same methods, a synthetic DNA having a codon ATC for isoleucine residue at the 30th position of a Factor X gene instead of codon GTC for the valine residue was prepared.

## Example 2

Synthesis of DNA fragments coding the amino acid residues including Gla domain of prothrombin, Factor VII or Factor IX

By almost the same methods described in Example 1, synthetic DNA fragments shown in Tables 3-5 were synthesized so as to flanked by SalI site and EcoRI site for convenience of ligation and to cover (a) the 40 amino acid residues of leader sequence of Factor X, (b) the lst to 43rd amino acid residues from the N-terminus of mature prothrombin, mature Factor VII, or mature Factor IX following the leader sequence, and (c) the nucleotide sequence coding the 44th to 46th amino acid residues of human protein C.

Example 3

Construction of expression vectors for hybrid proteins

The gene coding partial protein C could be removed from pCs4 by SalI digestion at the SalI site just upstream of leader sequence-coding sequence and at the SalI site corresponding to the 45th valine residue and 46th aspartic acid residue of protein C. The synthetic DNA fragment synthesized in Example 1 was inserted into pCs4 between these SalI sites, and introduced into E. coli K-12/HB101. The transformants harbouring a plasmid in an appropriate orientation, named pCs6, were screened and cultivated. pCs6 had the nucleotide sequence, shown in Table 6, of the hybrid protein between protein C and Factor X.

Using the same method, the synthetic DNA for Factor X whose 30th amino acid residue was substituted by isoleucine prepared in Example 1, and the synthetic DNA fragments prepared in Example 2 were combined for construction of an expression vector for another hybrid protein. The synthetic DNAs shown in Tables 3-5 are also employed to construct expression vectors of other hybrid proteins.

Example 4

Production of hybrid proteins in animal cells and confirmation of its protein C-like activities

After digesting pCs6, prepared in Example 3, by BstXI, a 2.0 Kbp DNA fragment was isolated by agarose gel electrophoresis. This 2.0 Kbp DNA and Bstxi-digested pHSG293 were ligated at the molar ratio of 20 and 1, respectively. This ligated DNA was packaged in vitro by lamda phage packaging mixture commercially available from Takara Shuzo Co., Ltd. Then packaged recombinant lamda genomes were transfected into E. coli K-12/Om206, deposited as FERM BP-1472 in Fermentation Research Institute. The transfectants were cultivated under the condition to select kanamycin resistants. The colonies harbouring high copy numbers of genes coding a hybrid protein were chosen and cultivated in the medium culture to isolate circular cosmid DNAs for expression of a hybrid protein.

These circular cosmid DNAs were introduced into CHO cells by a calcium·phosphate method. Then G418-resistant transfectants With pHSG293 were screened and cultured in MEMα medium, commecially available from Gibco Laboratories Inc., including 10% FCS, purchased from Gibco Laboratories Inc., and 0.1 μg/ml vitamin K₃.

About 1 x 10⁶ transfectants were seeded into a 6 cm-⌀ petri dish and cultivated at 37°C overnight. After exchanging the overnight medium for fresh medium, the cells were additionally cultivated for at least 24 hours. After collecting the culture supernatant, the amount of protein C-like antigen in the medium was assayed by ELISA using anti-human protein C antibody.

Consequently, five out of 20 clones were confirmed to produce 420 ng/ml, 530 ng/ml, 550 ng/ml, 910 ng/ml, or 1030 ng/ml of immunoreactive protein C in the medium. The best colony was further analyzed for its serine protease activity and anticoagulation activity by the human protein C assay kit, purchased from Yamanouchi Pharmaceutical Co., Ltd. (Cat. No. 917435 and 917567), and by the human protein C anticoagulation activity assay kit, purchased from Behringwerke AG, respectively. The result was 56% of serine protease activity and 62% of anticoagulation activity compared with human plasma as a control standard.

Example 5

Purification of a hybrid protein C

The CHO cells producing the hybrid protein C (PcGFX) constructed by replacing its Gla domain with Gla domain of Factor X (PcGFX) were cultivated with the dish culture method. In this example, 27.5 liter of culture supernatant was collected. PcGFX was purified by the method of Kisiel, J. Clin. Invest. 64, 761-769 (1979). The fraction absorbed in BaCl₂ from the supernatant was treated with 40% saturated ammonium sulfate for precipitation and then subjected to anti-human protein C monoclonal antibody affinity chromatography. PcGFX eluted from the affinity chromatography was more than 95% pure and the amount of protein was 4.85 mg.

The overall yield through this purification procedure was around 30%. Amino acid composition analysis revealed that expected 13 gla residues existed in PcGFX.

Example 6

Inactivation of Factor Va by PcGFX:

The effect of PcGFX on Factor Va, a key serine protease in blood coagulation cascade, which was known to be inactivated by protein C in vivo was assayed by measuring in vitro platelet-thrombin converting activity of Factor Va in the presence of either PcGFX or natural human protein C, as a control standard. PcGFX and natural protein C were activated by Protac®, described in Thromb. Res. 43, 253-264, (1986). As shown in the following table, 0.2 unit/ml of PcGFX had more potent inhibitory activity against the thrombin generation by Factor Va compared with that of natural protein C (n-protein C in the following table).

| Incubation time (min) | Thrombin generation (IU/ml) | | |
|---|---|---|---|
| | Control | n-protein C (0.2 U/ml) | r-PcGFX* (0.2 U/ml) |
| 0 | 21.1 | 14.3 | 13.4 |
| 5 | 21.8 | 14.0 | 6.1 |
| 10 | 21.8 | 8.3 | 3.5 |
| 20 | 21.3 | 4.4 | 2.5 |

* Recombinant PcGFX

Example 7

Inactivation of Factor VIIIc by PcGFX

The effect of PcGFX on Factor VIIIc was assayed by TESTZYM® FVIII kit, commercially available from Daiichi Pure Chemicals Co., Ltd. Activation of PcGFX and natural protein C was done by the same method as described in Example 6. Activated PcGFX or natural protein C was mixed with Factor VIIIc and was incubated for 0, 15, 30, 45, and 60 min, and then the reaction mixtures were diluted 1:121, mixed additionally with phospholipids, Factor IXa, Factor X and CaCl₂, and were incubated for at least 5 min at 37° C.

After the incubation, S-2222, obtained commercially from AB Kabivitrum (Sweden), was added as a substrate of Factor Xa produced in the reaction mixture. The production of Factor Xa was measured as inhibitory activity against Factor VIIIc by PcGFX or natural protein C. The result in the following table shows that the inhibitory activity of PcGFX against Factor VIIIc was almost the same as that of natural protein C.

| Incubation time (min) | Factor Xa generation (%) | | |
|---|---|---|---|
| | Control | n-protein C | r-PcGFX* |
| 0 | 100.0 | 100.0 | 100.0 |
| 15 | 87.1 | 19.6 | 26.7 |
| 30 | 78.9 | 5.5 | 5.5 |
| 45 | 78.0 | 2.1 | 1.5 |
| 60 | 76.8 | 0.0 | 0.0 |

* Recombinant PcGFX

Explanation of Tables

Table 1 shows alignments of the amino acid sequence near the amino terminus of prothrombin, Factor VII, Factor IX, Factor X, and protein C.

Table 2 shows the nucleotide sequence of synthetic DNA coding amino acid sequecne including Gla domain of Factor X.

Table 3 shows the nucleotide sequence of synthetic DNA coding amino acid sequence including Gla domain of prothrombin.

Table 4 shows the nucleotide sequence of synthetic DNA coding amino acid sequence including Gla domain of Factor VII.

Table 5 shows the nucleotide sequence of synthetic DNA coding amino acid sequence including Gla of Factor IX.

Table 6 shows the nucleotide sequence coding hybrid protein constructed by replacing Gla domain of protein C by Gla domain of Factor X, and the amino acid sequence of the hybrid protein.

EP 0 296 413 A2

## Table 1

|  | 10 | 20 | 30 | 40 |
|---|---|---|---|---|
| Prothrombin | ANT*FLEEVRKGNLERECVEETCSYEEAFEALESSTATDVFWAKY |
| Factor VII | ANA*FLEELRPGSLERECKEEQCSFEEAREIFKDAERTKLFWISY |
| Factor IX | YNSGKLEEFVQGNLERECMEEKCSFEEAREVFENTEKTTEFWKQY |
| Factor X | ANS*FLEEMKKGHLERECMEETCSYEEAREVFEDSDKTNEFWNKY |
| Protein C | ANS*FLEELRHSSLERECIEEICDFEEAKEIFQNVDDTLAFWSKH |

## Table 2

┌→ Leader sequence of Factor X

<u>GTCGACGAAT TCA</u>ATGGGGC GCCCACTGCA CCTCGTCCTG CTCAGTGCCT CCCTGGCTGG

Saℓ 1     EcoR 1

CCTCCTGCTG CTCGGGGA<u>AA GCTT</u>GTTCAT CCGCAGGGAG CAGGCCAACA ACATCCTGGC

Hind Ⅲ

┌→ 1-43 amino acid residues of Factor X

GAGGGTCACG AGGGCCAATT CCTT<u>TCTAGA</u> AGAGATGAAG AAAGGACACC TCGAAAGAGA

Xba 1

GTGCATGGAA GAGAC<u>CTGCA G</u>CTACGAAGA GGCCCGCGAG GTCTTTGAGG ACAGCGACAA

Pst 1

┌→ 44-46 amino acid residues of protein C

GACGAATGAG TTCTGGAATA AACAC<u>GTCGA C</u>

Saℓ 1

EP 0 296 413 A2

## Table 3

Leader sequence of Factor X

<u>GTCGACGAAT TCA</u>ATGGGGC GCCCACTGCA CCTCGTCCTG CTCAGTGCCT CCCTGGCTGG

Sal I   EcoR I

CCTCCTGCTG CTCGGGGA<u>AA GCTT</u>GTTCAT CCGCAGGGAG CAGGCCAACA ACATCCTGGC

Hind Ⅲ

1-43 amino acid residues of prothrombin

GAGGGTCACG AGGGCCAACA CCTTCTTGGA GGAGG<u>TGCGC A</u>AGGGCAACC TAGAGCGAGA

Mst I

GTGCGTGGAG GAGACGTGCA GCTACGAGG<u>A GGCCT</u>TCGAG GCTCTGGAGT CCTCCACGGC

Stu I

44-46 amino acid residues of protein C

TACGGATGTG TTCTGGGCCA AGCAC<u>GTCGA C</u>

Sal I

EP 0 296 413 A2

## Table 4

**⌐→ Leader sequence of Factor X**
GTCGACGAAT TCAATGGGGC GCCCACTGCA CCTCGTCCTG CTCAGTGCCT CCCTGGCTGG
  Saℓ 1    EcoR 1

CCTCCTGCTG CTCGGGGAAA GCTTGTTCAT CCGCAGGGAG CAGGCCAACA ACATCCTGGC
          Hind III

**⌐→ 1-43 amino acid residues of Factor VII**
GAGGGTCACG AGGGCCAACG CGTTCCTGGA GGAGCTGCGG CCGGGCTCCC TGGAGAGGGA
                                      Xma III

GTGCAAGGAG GAGCAGTGCT CCTTCGAGGA GGCCCGGGAG ATCTTCAAGG ACGCGGAGAG
                              Xma I

**⌐→ 44-46 amino acid residues of protein C**
GACGAAGCTG TTCTGGATTT CTCACGTCGA C
                      Saℓ 1

EP 0 296 413 A2

## Table 5

┌─ Leader sequence of Factor X

<u>GTCGACGAAT</u> TCA|ATGGGGC GCCCACTGCA CCTCGTCCTG CTCAGTGCCT CCCTGGCTGG

  Sa*l* 1    EcoR 1


CCTCCTGCTG CTCGGGGA<u>AA GCTT</u>GTTCAT CCGCAGGGAG CAGGCCAACA ACATCCTGGC

               H Ind Ⅲ


┌→1-43 amino acid residues of Factor IX

GAGGGTCACG AGG|TATAATT CAGGTAAATT GGAAGAGTTT GTTCAAGGGA A<u>TCTAGA</u>GAG

                                                                             Xba 1


AGAATGTATG GAAGAAAAGT GTAGTTTTGA AGAAGCACGA GAAGTTTTTG AAAACACTGA


┌→44-46 amino acid residues of protein C

AAAGACAACT GAATTTTGGA AG|CACG<u>T CGAC</u>

                        Sa*l* 1

EP 0 296 413 A2

EP 0 296 413 A2

## Table 6

```
-40                                                    -30
ATG. GGG. CGC. CCA. CTG. CAC. CTC. GTC. CTG. CTC. AGT. GCC. TCC. CTG. GCT. GGC. CTC. CTG. CTG. CTC
Met-Gly-Arg-Pro-Leu-His-Leu-Val-Leu-Leu-Ser-Ala-Ser-Leu-Ala-Gly-Leu-Leu-Leu-Leu


-20                                                    -10
GGG. GAA. AGC. TTG. TTC. ATC. CGC. AGG. GAG. CAG. GCC. AAC. AAC. ATC. CTG. GCG. AGG. GTC. ACG. AGG
Gly-Glu-Ser-Leu-Phe-Ile-Arg-Arg-Glu-Gln-Ala-Asn-Asn-Ile-Leu-Ala-Arg-Val-Thr-Arg


1                                                      10                                               20
GCC. AAT. TCC. TTT. CTA. GAA. GAG. ATG. AAG. AAA. GGA. CAC. CTC. GAA. AGA. GAG. TGC. ATG. GAA. GAG
Ala-Asn-Ser-Phe-Leu-Glu-Glu-Met-Lys-Lys-Gly-His-Leu-Glu-Arg-Glu-Cys-Met-Glu-Glu


                                                       30                                               40
ACC. TGC. AGC. TAC. GAA. GAG. GCC. CGC. GAG. GTC. TTT. GAG. GAC. AGC. GAC. AAG. ACG. AAT. GAG. TTC
Thr-Cys-Ser-Tyr-Glu-Glu-Ala-Arg-Glu-Val-Phe-Glu-Asp-Ser-Asp-Lys-Thr-Asn-Glu-Phe


                                                       50                                               60
TGG. AAT. AAA. CAC. GTC. GAC. GGT. GAC. CAG. TGC. TTG. GTC. TTG. CCC. TTG. GAG. CAC. CCG. TGC. GCC
Trp-Asn-Lys-His-Val-Asp-Gly-Asp-Gln-Cys-Leu-Val-Leu-Pro-Leu-Glu-His-Pro-Cys-Ala


                                                       70                                               80
AGC. CTG. TGC. TGC. GGG. CAC. GGC. ACG. TGC. ATC. GAT. GGC. ATC. GGC. AGC. TTC. AGC. TGC. GAC. TGC
Ser-Leu-Cys-Cys-Gly-His-Gly-Thr-Cys-Ile-Asp-Gly-Ile-Gly-Ser-Phe-Ser-Cys-Asp-Cys
```

EP 0 296 413 A2

90
CGC. AGC. GGC. TGG. GAG. GGC. CGC. TTC. TGC. CAG. CGC. GAG. GTA. AGC. TTC. CTC. AAT. TGC. TCT. CTG
Arg-Ser-Gly-Trp-Glu-Gly-Arg-Phe-Cys-Gln-Arg-Glu-Val-Ser-Phe-Leu-Asn-Cys-Ser-Leu

110
GAC. AAC. GGC. GGC. TGC. ACG. CAT. TAC. TGC. CTA. GAG. GAG. GTG. GGC. TGG. CGG. CGC. TGT. AGC. TGT
Asp-Asn-Gly-Gly-Cys-Thr-His-Tyr-Cys-Leu-Glu-Glu-Val-Gly-Trp-Arg-Arg-Cys-Ser-Cys

130
GCG. CCT. GGC. TAC. AAG. CTG. GGG. GAC. GAC. CTC. CTG. CAG. TGT. CAC. CCC. GCA. GTG. AAG. TTC. CCT
Ala-Pro-Gly-Tyr-Lys-Leu-Gly-Asp-Asp-Leu-Leu-Gln-Cys-His-Pro-Ala-Val-Lys-Phe-Pro

150
TGT. GGG. AGG. CCC. TGG. AAG. CGG. ATG. GAG. AAG. AAG. AGA. TCT. CAC. CTG. AAA. CGA. GAC. ACA. GAA
Cys-Gly-Arg-Pro-Trp-Lys-Arg-Met-Glu-Lys-Lys-Arg-Ser-His-Leu-Lys-Arg-Asp-Thr-Glu

170
GAC. CAA. GAA. GAC. CAA. GTA. GAT. CCG. CGG. CTC. ATT. GAT. GGG. AAG. ATG. ACC. AGG. CGG. GGA. GAC
Asp-Gln-Glu-Asp-Gln-Val-Asp-Pro-Arg-Leu-Ile-Asp-Gly-Lys-Met-Thr-Arg-Arg-Gly-Asp

190
AGC. CCC. TGG. CAG. GTG. GTC. CTT. CTA. GAC. TCA. AAG. AAG. AAG. CTG. GCC. TGC. GGG. GCA. GTG. CTC
Ser-Pro-Trp-Gln-Val-Val-Leu-Leu-Asp-Ser-Lys-Lys-Lys-Leu-Ala-Cys-Gly-Ala-Val-Leu

EP 0 296 413 A2

210                                                                              220
ATC. CAC. CCC. TCC. TGG. GTG. CTG. ACT. GCA. GCC. CAC. TGC. ATG. GAT. GAG. TCC. AAG. AAG. CTC. CTT
Ile-His-Pro-Ser-Trp-Val-Leu-Thr-Ala-Ala-His-Cys-Met-Asp-Glu-Ser-Lys-Lys-Leu-Leu

230                                                                              240
GTC. AGG. CTT. GGA. GAG. TAT. GAC. CTG. CGG. CGC. TGG. GAG. AAG. TGG. GAG. CTG. GAC. CTG. GAC. ATC
Val-Arg-Leu-Gly-Glu-Tyr-Asp-Leu-Arg-Arg-Trp-Glu-Lys-Trp-Glu-Leu-Asp-Leu-Asp-Ile

250                                                                              260
AAG. GAG. GTC. TTC. GTC. CAC. CCC. AAC. TAC. AGC. AAG. AGC. ACC. ACC. GAC. AAT. GAC. ATC. GCA. CTG
Lys-Glu-Val-Phe-Val-His-Pro-Asn-Tyr-Ser-Lys-Ser-Thr-Thr-Asp-Asn-Asp-Ile-Ala-Leu

270                                                                              280
CTG. CAC. CTG. GCC. CAG. CCC. GCC. ACC. CTC. TCG. CAG. ACC. ATA. GTG. CCC. ATC. TGC. CTC. CCG. GAC
Leu-His-Leu-Ala-Gln-Pro-Ala-Thr-Leu-Ser-Gln-Thr-Ile-Val-Pro-Ile-Cys-Leu-Pro-Asp

290                                                                              300
AGC. GGC. CTT. GCA. GAG. CGC. GAG. CTC. AAT. CAG. GCC. GGC. CAG. GAG. ACC. CTC. GTG. ACG. GGC. TGG
Ser-Gly-Leu-Ala-Glu-Arg-Glu-Leu-Asn-Gln-Ala-Gly-Gln-Glu-Thr-Leu-Val-Thr-Gly-Trp

310                                                                              320
GGC. TAC. CAC. AGC. AGC. CGA. GAG. AAG. GAG. GCC. AAG. AGA. AAC. CGC. ACC. TTC. GTC. CTC. AAC. TTC
Gly-Tyr-His-Ser-Ser-Arg-Glu-Lys-Glu-Ala-Lys-Arg-Asn-Arg-Thr-Phe-Val-Leu-Asn-Phe

```
                                          330                                           340
ATC. AAG. ATT. CCC. GTG. GTC. CCG. CAC. AAT. GAG. TGC. AGC. GAG. GTC. ATG. AGC. AAC. ATG. GTG. TCT
Ile-Lys-Ile-Pro-Val-Val-Pro-His-Asn-Glu-Cys-Ser-Glu-Val-Met-Ser-Asn-Met-Val-Ser

                                          350                                           360
GAG. AAC. ATG. CTG. TGT. GCG. GGC. ATC. CTC. GGG. GAC. CGG. CAG. GAT. GCC. TGC. GAG. GGC. GAC. AGT
Glu-Asn-Met-Leu-Cys-Ala-Gly-Ile-Leu-Gly-Asp-Arg-Gln-Asp-Ala-Cys-Glu-Gly-Asp-Ser

                                          370                                           380
GGG. GGG. CCC. ATG. GTC. GCC. TCC. TTC. CAC. GGC. ACC. TGG. TTC. CTG. GTG. GGC. CTG. GTG. AGC. TGG
Gly-Gly-Pro-Met-Val-Ala-Ser-Phe-His-Gly-Thr-Trp-Phe-Leu-Val-Gly-Leu-Val-Ser-Trp

                                          390                                           400
GGT. GAG. GGC. TGT. GGG. CTC. CTT. CAC. AAC. TAC. GGC. GTT. TAC. ACC. AAA. GTC. AGC. CGC. TAC. CTC
Gly-Glu-Gly-Cys-Gly-Leu-Leu-His-Asn-Tyr-Gly-Val-Tyr-Thr-Lys-Val-Ser-Arg-Tyr-Leu

                                          410                                           420
GAC. TGG. ATC. CAT. GGG. CAC. ATC. AGA. GAC. AAG. GAA. GCC. CCC. CAG. AAG. AGC. TGG. GCA. CCT. TAG
Asp-Trp-Ile-His-Gly-His-Ile-Arg-Asp-Lys-Glu-Ala-Pro-Gln-Lys-Ser-Trp-Ala-Pro-Ter

TAA
Ter


        Ter is a termination codon
```

EP 0 296 413 A2

## Claims

1. A hybrid protein of human protein C whose amino acid sequence from the lst to 43rd residues is substituted with an amino acid sequence from the lst to 43rd residues of human prothrombin, Factor VII, Factor IX, or Factor X.

2. A hybrid protein as claimed in Claim 1 wherein the 30th valine residue of Factor X is further substituted with isoleucine.

3. DNA coding a hybrid protein of human protein C whose amino acid sequence from the lst to 43rd residues of human protein C is substituted with an amino acid sequence from the lst to 43rd residues of human prothrombin, Factor VII, Factor IX, or Factor X.

4. DNA as claimed in Claim 3 coding a hybrid protein wherein the 30th valine residue of human Factor X is further substituted with isoleucine.

5. A method for producing a hybrid protein which employs DNA coding a hybrid protein of human protein C whose amino acid sequence from the lst to 43rd residues of human protein C is substituted with an amino acid sequence from the lst to 43rd residues of human prothrombin, Factor VII, Factor IX, or Factor X.

6. A method for producing a hybrid protein as claimed in Claim 5 which employs DNA coding a hybrid protein wherein the 30th valine residue of Factor X is further substituted with isoleucine.

Claims for the following Contracting States: ES, GR

1. A method for producing a hybrid protein which employs DNA coding a hybrid protein of human protein C whose amino acid sequence from the lst to 43rd residues of human protein C is substituted with an amino acid sequence from the lst to 43rd residues of human prothrombin, Factor VII, Factor IX, or Factor X.

2. A method for producing a hybrid protein as claimed in Claim 5 which employs DNA coding a hybrid protein wherein the 30th valine residue of Factor X is further substituted with. isoleucine.

FIG.1

FIG. 2

FIG.3